# EUROPÄISCHE PATENTSCHRIFT

(11) **EP 2 190 386 B1**
(45) Veröffentlichungstag und Bekanntmachung des Hinweises auf die Patenterteilung: **20.04.2011**
(21) Anmeldenummer: 08785431.1
(22) Anmeldetag: 07.08.2008
(51) Int. Cl.: A61F 2/30

(54) **ENDOPROTHESENKOMPONENTE**
ENDOPROSTHESIS COMPONENT
ÉLÉMENT ENDOPROTHÉTIQUE

(30) Priorität: 17.09.2007 EP 07018225
(43) Veröffentlichungstag der Anmeldung: 02.06.2010
(73) Patentinhaber: WALDEMAR LINK GmbH & Co. KG, 22339 Hamburg (DE)
(72) Erfinder: THULL, Roger, 97082 Würzburg (DE); LINK, Helmut, D., 22397 Hamburg (DE)
(74) Vertreter: Glawe, Delfs, Moll
(86) Internationale Anmeldenummer: PCT/EP2008/006525
(87) Internationale Veröffentlichungsnummer: WO 2009/036846

(56) Entgegenhaltungen:
- WO-A-03/094774
- US-A- 5 674 280
- US-A1- 2006 051 392

## Beschreibung

Die Erfindung betrifft eine Endoprothesenkomponente mit einem die Form der Endoprothesenkomponente vorgebenden Körper. Auf Oberflächenanteilen, mit denen die Endoprothesenkomponente im implantierten Zustand Kontakt mit menschlichem Gewebe eingeht, ist der Körper mit einer Außenschicht belegt. Die Außenschicht umfasst ein Nitrid, ein Oxinitrid oder ein Oxid auf der Basis eines Refraktärmetalls, ferner ist in der Außenschicht Silber und/oder Kupfer enthalten. Die Erfindung betrifft ferner ein Verfahren zur Herstellung einer solchen Endoprothesenkomponente.

Endoprothesenkomponenten, die Bestandteile des menschlichen Skeletts ersetzen, müssen mehrere Anforderungen erfüllen. Die Endoprothesenkomponente muss hinreichend stabil sein, damit sie die im Skelett auftretenden Kräfte aufnehmen kann. Außerdem müssen die Elemente der Endoprothesenkomponente, die in implantiertem Zustand Kontakt mit Gewebe, was auch Knochenmaterial umfasst, des menschlichen Körpers eingehen, eine gute Verträglichkeit aufweisen.

Bereits bekannt ist es, Oberflächenanteile der Endoprothesenkomponente, die für Kontakt mit menschlichem Gewebe ausgelegt sind, mit einer Schicht aus einem harten Material, wie beispielsweise einem Nitrid, einem Oxinitrid oder einem Oxid auf der Basis eines Refraktärmetalls zu belegen. Hartstoffbeschichtungen dieser Art verbinden gute mechanische Eigenschaften mit einer guten Verträglichkeit.

Dennoch kommt es bei der Annahme derartiger Endoprothesenkomponente durch den menschlichen Körper gelegentlich zu Problemen. Es kann nämlich passieren, dass sich auf der Oberfläche der Endoprothesenkomponente Mikroorganismen festsetzen, die die Aufnahme der Endoprothesenkomponente in den menschlichen Körper behindern. Die Gefahr durch Mikroorganismen auf der Oberfläche von Endoprothesenkomponenten ist je größer, desto größer der Eingriff ist, der zum Einsetzen der Endoprothesenkomponente in den Körper erforderlich ist, und je größer die Fläche ist, über die die Endoprothesenkomponente mit dem Gewebe des Körpers in Berührung kommt. Operationen, bei denen ein großer Eingriff erforderlich ist und bei denen die Endoprothese großflächig mit Gewebe des menschlichen Körpers in Kontakt gebracht wird, sind beispielsweise Kopf-Schaft-Prothesenschäfte oder solche, bei denen der Oberschenkelknochen komplett durch eine Endoprothese ersetzt wird.

Aus WO 03/094774 ist es bekannt, die Oberfläche eines aus Metall oder einer Metalllegierung gebildeten Implantats zu oxidieren und die entstehende Hartstoffschicht mit Silberpartikeln zu versehen, um eine antimikrobielle Wirkung zu erzielen. Da Hartstoffschichten nur sehr langsam mit dem umgebenden Körpergewebe reagieren, werden die Silberpartikel über einen langen Zeitraum aus der Hartstoffschicht heraus abgegeben. Eine solche Hartstoffbeschichtung kann also eine gute Langzeitwirkung bieten. Unmittelbar nach dem Einsetzen des Implantats kann sich jedoch eine größere Zahl von Mikrorganismen in der Umgebung des Implantats finden. Die aus der Hartstoffschicht abgegebene Menge an Silberpartikeln ist zu gering, um gegen eine größere Anzahl von Mikroorganismen wirksam zu sein.

Der Erfindung liegt die Aufgabe zu Grunde, eine Endoprothesenkomponente der eingangs genannten Art sowie ein Verfahren zur Herstellung einer solchen Endoprothesenkomponente vorzustellen, bei denen die Wahrscheinlichkeit, dass die Aufnahme der Endoprothesenkomponente in den menschlichen Körper durch Mikroorganismen behindert wird, vermindert ist. Die Aufgabe wird gelöst durch die Merkmale der unabhängigen Ansprüche. Vorteilhafte Ausführungsformen finden sich in den Unteransprüchen. Die erfindungsgemäße Endoprothesenkomponente zeichnet sich dadurch aus, dass zwischen der Außenschicht und dem Körper eine Zwischenschicht vorgesehen ist und dass Teile der Zwischenschicht von außen zugänglich sind.

Zunächst werden einige Begriffe erläutert. Ein Körper, der die Form der Endoprothesenkomponente vorgibt, unterscheidet sich zumindest in dem Bereich, in dem die Oberfläche der Endoprothesenkomponente für einen Kontakt mit menschlichem Gewebe ausgelegt ist, nur durch zusätzlich auf die Oberfläche aufgebrachte Beschichtungen von der Form der Endoprothesenkomponente.

Regelmäßig ist nicht die gesamte Oberfläche der Endoprothesenkomponente dazu bestimmt, eine Verbindung mit menschlichem Gewebe einzugehen. Vielmehr kann die Endoprothesenkomponente Bereiche an ihrer Oberfläche umfassen, die dazu ausgelegt sind, beispielsweise mit einer weiteren Endoprothesenkomponente zusammenzuwirken, etwa um ein Gelenk zu bilden. Im Rahmen der Erfindung ist es nicht erforderlich, dass der gesamte Bereich der Oberfläche, der für Kontakt mit menschlichem Gewebe ausgelegt ist, mit einer Außenschicht oder einer Zwischenschicht belegt ist. Ausreichen kann es vielmehr, wenn Oberflächenanteile dieser Bereiche mit einer Außenschicht und/oder einer Zwischenschicht belegt sind.

Als Außenschicht wird eine Schicht bezeichnet, die unmittelbaren Kontakt zur Umgebung hat. Eine Außenschicht ist also nicht mit einer weiteren Schicht belegt. Die Außenschicht muss nicht flächendeckend sein, sie kann auch Durchbrechungen aufweisen oder lediglich inselförmig aufgebracht sein.

Eine Zwischenschicht ist zumindest auf Teilen ihrer Oberfläche mit einer Außenschicht bedeckt. In Bereichen, in denen keine Außenschicht aufgebracht ist oder in denen die Außenschicht Durchbrechnungen aufweist, hat die Zwischenschicht selbst unmittelbaren Kontakt zur Umgebung. Die Zwischenschicht ist also teilweise von außen zugänglich. Auf der Oberfläche der Endoprothesenkomponente wechseln sich Stellen, an denen die Zwischenschicht von außen zugänglich ist, ab mit Stellen, an den die Zwischenschicht nicht von außen zugänglich ist. Die Zwischenschicht besteht aus einem anderen Material als dem Material, aus dem die Endoprothesenkomponente geformt ist.

Der Begriff menschliches Gewebe ist umfassend zu verstehen. Er umfasst alle Elemente des menschlichen Körpers, mit denen eine Endoprothese in Berührung kommen kann, also beispielsweise auch Knochen.

Refraktärmetalle sind hochschmelzende, unedle Metalle der vierten, fünften und sechsten Nebengruppe. Umfasst sind in der vierten Nebengruppe Titan, Zirconium und Hafnium, in der fünften Nebengruppevanadium, Niob und Tantal sowie in der sechsten Nebengruppe Chrom, Molybdän und Wolfram. Für die Beschichtung von Endoprothesenkomponenten besonders geeignete Refraktärmetalle sind Titan, Zirkonium, Niob und Tantal. Als Nitrid, Oxinitrid oder Oxid auf der Basis eines Refraktärmetalls werden Verbindungen bezeichnet, die Ionen eines Refraktärmetalls mit Sauerstoff und/oder Stickstoff als reaktivem Gas eingehen. Diese Verbindungen zeichnen sich durch eine große Härte aus, die entstehende Schicht wird deswegen auch als Hartstoffschicht bezeichnet.

Wenn die Außenschicht zusätzlich Silber und/oder Kupfer umfasst, so sind bei der Entstehung des Nitrids, Oxinitrids bzw. Oxids nicht nur Ionen des Refraktärmetalls und ein reaktives Gas, sondern zusätzlich Silber- und/oder Kupferionen beteiligt. Die Silber- und/oder Kupferionen werden in die entstehende Beschichtung integriert. Die Außenschicht hat durch das enthaltene Silber und/oder Kupfer eine antimikrobielle Langzeitwirkung. Die antimikrobielle Wirkung ist mit guten mechanischen Eigenschaften wie großer Härte und hoher Abriebfestigkeit verbunden.

Indem zwischen der Außenschicht und dem Körper eine Zwischenschicht vorgesehen ist, die teilweise von außen zugänglich ist, wird es möglich, die Oberfläche der Endoprothesenkomponente außer der antimikrobiellen Langzeitwirkung der Hartstoffbeschichtung mit weiteren Wirkungen zu versehen. So kann beispielsweise die Zwischenschicht so ausgebildet sein, dass sie in kurzer Zeit eine höhere Dosis eines antimikrobiellen Wirkstoffs abgibt. Die höhere Dosis wirkt dann unmittelbar im Anschluss an die Operation gegen Infektionen.

Der Körper, der die Form der Endoprothesenkomponente vorgibt, kann aus Metall oder aus einer Metalllegierung bestehen. Besonders geeignete Materialien sind Titan und Titanlegierungen. Der Begriff Außenschicht umfasst auch eine Schicht, die in mehreren Auftragdurchgängen erzeugt wurde. Das Aufbringen einer Schicht in mehreren Auftragdurchgängen kommt insbesondere dann in Betracht, wenn die in einem Auftragdurchgang erzeugte Schicht zu dünn ist.

Die Zwischenschicht kann eine Schicht aus Silber, Kupfer und/oder Gold sein. Vorzugsweise ist die Zwischenschicht flächendeckend. Eine solche Zwischenschicht korrodiert in Kontakt mit menschlichem Gewebe wesentlich schneller als das Hartstoffmaterial der Außenschicht. Es wird also eine größere Dosis von antimikrobiell wirksamen Partikeln abgegeben.

In Bereichen, in denen die Zwischenschicht von außen zugänglich ist, ist die Außenschicht durchbrochen. Die Durchbrechungen können Durchmesser von lediglich einigen µm haben oder wesentlich größer sein. Bei mikroskopischen Durchbrechungen mit einem Mindestdurchmesser von lediglich einigen µm spricht man von einer porösen Außenschicht. Makroskopische Durchbrechungen mit Durchmessern beispielsweise im mm-Bereich ermöglichen einen größerflächigen Zugang zur Zwischenschicht von außen. Insbesondere kann die Außenschicht so gestaltet sein, dass die Durchbrechungen einen Flächenanteil von mindestens 20%, vorzugsweise mindestens 40%, weiter vorzugsweise mindestens 60% einnehmen.

Die antimikrobielle Wirkung kann verstärkt werden, wenn die Außenschicht eine Mikro- oder Nanostruktur mit ausgeprägten Spitzen hat. Eine ausgeprägte Spitze in einer Oberflächenstruktur bezeichnet eine Formation, bei der die Oberfläche ausgehend von einem Extremum in allen Richtungen mit einem Winkel von mehr als 45° bezogen auf die Richtung der Oberfläche abfällt. Elektrisch leitfähige Spitzen bilden ein im Vergleich zu glatten Oberflächen starkes elektrisches Feld in ihrer Umgebung aus, das eine negative Wirkung auf Mikroorganismen ausübt.

Die Erfindung betrifft ferner ein Verfahren zum Herstellen einer solchen Endoprothesenkomponente. Zum Herstellen der Endoprothesenkomponente wird zunächst ein Körper bereitgestellt, der die Form der Endoprothesenkomponente vorgibt. Oberflächenanteile des Körpers, die für eine Verbindung mit menschlichem Gewebe ausgelegt sind, sind mit einer Beschichtung versehen, die zwar zu diesem Zeitpunkt noch nicht mit einer Außenschicht belegt ist, die aber der Einfachheit halber trotzdem als Zwischenschicht bezeichnet wird. An die Oberflächenstruktur der Zwischenschicht werden keine besonderen Anforderungen gestellt. Die Oberfläche kann also glatt, makro-, mikro- oder nanostrukturiert sein.

Ferner werden Targets bereitgestellt, aus denen das Material, das später die Beschichtung bilden soll, herausgelöst werden kann. Es kann ein einzelnes Target bereitgestellt werden, das sowohl das Refraktärmetall als auch das Silber und/oder Kupfer umfasst. Möglich ist es aber auch, mehrere Targets bereitzustellen, wobei ein erstes Target ein Refraktärmetall umfasst und wobei ein zweites Target Silber und/oder Kupfer umfasst.

Aus dem einen oder den mehreren Targets werden Ionen des Refraktärmetalls sowie Ionen von Silber und/oder Kupfer herausgelöst. Zum Herauslösen der Ionen kann beispielsweise ein Lichtbogen zwischen einer Elektrode und dem Target erzeugt werden, der dem Target lokal begrenzt soviel Energie zuführt, dass Ionen herausgelöst werden. Eine andere Möglichkeit, dem Target lokal ausreichend Energie zuzuführen, kann darin bestehen, einen Laserstrahl auf das Target zu richten. Alternativ, wenn auch sehr teuer, ist die Energiezufuhr auch durch einen Elektronenstrahl möglich.

Die freien Ionen werden zusammen mit einem reaktiven Gas derart auf mit der Zwischenschicht belegte Oberflächenanteile des Körpers geleitet, dass sich eine Außenschicht bildet, durch die hindurch die Zwischenschicht zugänglich ist. Dazu kann eine Spannung zwischen das Target und den Körper gelegt werden, durch die die Ionen in Richtung des Körpers beschleunigt werden. Das Reaktionsgefäß mit Target und Körper enthält ein reaktives Gas, wie beispielsweise Sauerstoff oder Stickstoff oder ein Gemisch von Sauerstoff und Stickstoff. Die Ionen des Refraktärmetalls sowie die Ionen von Silber und/oder Kupfer reagieren mit dem reaktiven Gas und es bilden sich Moleküle, die alle drei Bestandteile enthalten. In einer Ausführungsform bilden sich Ti-AgN-Moleküle. Durch die auf der Oberfläche des Körpers stattfindende Reaktion entsteht die Außenschicht. Die Reaktion kann unter Vakuum, vorzugsweise unter Hochvakuum stattfinden. Verfahren dieser Art sind unter der Bezeichnung Plasma-Beschichtungsverfahren bekannt. Ein verbesserter Halt einer aufgebrachten Schicht kann erreicht werden, wenn die Oberfläche des Körpers zuvor durch Sandstrahlen behandelt wird.

Es gibt mehrere Möglichkeiten, mit denen sichergestellt werden kann, dass die Außenschicht sich so bildet, dass die Zwischenschicht zugänglich bleibt. Beispielsweise kann eine poröse Außenschicht mit mikroskopischen Durchbrechungen dadurch erzeugt werden, dass dem einzelnen oder den mehreren Targets soviel Energie zugeführt wird, dass sich außer den Ionen auch noch Bruchstücke aus den Targets herauszulösen, die einen größten Durchmesser von mehr als 20 µm haben. Die Bruchstücke bewegen sich statistisch in alle Richtungen und treffen dabei auch auf die Oberfläche des Körpers auf, auf der sich gerade die Außenschicht bildet. An den Stellen, an denen die Bruchstücke auftreffen, wird ein Stück aus der sich bildenden Außenschicht herausgeschlagen. Es entsteht eine poröse Außenschicht, durch die hindurch die Zwischenschicht zugänglich ist.

Eine andere Möglichkeit, die Zugänglichkeit der Zwischenschicht zu gewährleisten, besteht darin, den zu beschichtenden Oberflächenanteil des Körpers mit einer Maske aus einem temperaturstabilen Material zu belegen. Die Maske belegt Teile der Oberfläche und lässt andere Teile der Oberfläche frei. Die Maske kann beispielsweise eine netzartige oder eine gitterartige Form haben. Die Außenschicht bildet sich dann inselförmig nur an den Stellen aus, die von der Maske freigelassen werden. Nach dem Entfernen des temperaturstabilen Materials wird die Zwischenschicht entsprechend der Maske zugänglich. Als temperaturstabil wird ein Material bezeichnet, das gegenüber den bei Plasma-Beschichtungsverfahren bestehenden Bedingungen beständig ist. Das temperaturstabile Material kann insbesondere ein Metall sein.

Werden die Parameter des Plasma-Beschichtungsverfahrens geeignet gewählt, so entsteht eine Außenschicht, die eine Mikro- oder Nanostruktur mit ausgeprägten Spitzen aufweist. Ist die Zwischenschicht aus Silber, Kupfer und/oder Gold, so kann sie ebenfalls mittels eines Plasma-Beschichtungsverfahrens aufgebracht werden. Alternativ ist es möglich, die Zwischenschicht mittels elektrochemischer Abscheidung auf den Körper aufzubringen.

Die Erfindung wird nachfolgend unter Bezugnahme auf die beigefügten Zeichnungen anhand einer vorteilhaften Ausführungsform beispielhaft beschrieben. Es zeigen:
- Fig. 1: eine mehrere Endoprothesenkomponenten umfassende Endoprothese;
- Fig. 2: eine erfindungsgemäße Endoprothesenkomponente;
- Fig. 3: ein vergrößerter Ausschnitt aus einem Querschnitt durch Fig. 2;
- Fig. 4: die Ansicht aus Fig. 3 bei einer anderen Ausfüh- rungsform der Erfindung;
- Fig. 5: die Ansicht aus Fig. 3 bei einer weiteren Ausfüh- rungsform der Erfindung; und
- Fig. 6: ein vergrößerter Ausschnitt aus der Oberfläche der Endoprothesenkomponente gemäß Fig. 2 bei einer Ausführungsform der Erfindung.

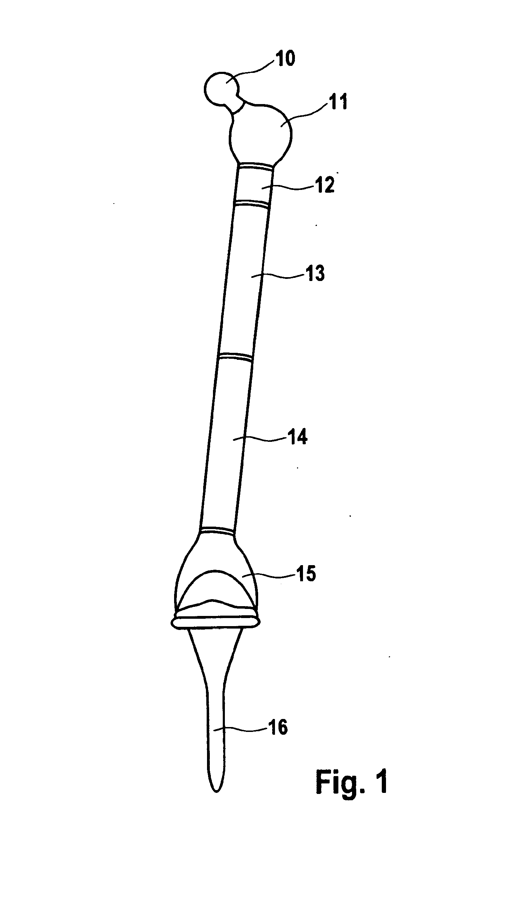

Eine in Fig. 1 gezeigte Endoprothese ist dazu bestimmt, einen von der Hüfte bis unterhalb des Knies reichenden Teil des menschlichen Skeletts zu ersetzen. Ein kugelförmiger Gelenkkopf 10 bildet eine Gelenkfläche, die dazu ausgelegt ist, mit einem Acetabulum zusammenzuwirken. Der Gelenkkopf 10 ist mit einem Kopfstück 11 der Endoprothese über eine Schraubverbindung verbunden. Der den Mittelschaft des Oberschenkelknochens ersetzende Teil der Endoprothese umfasst drei Endoprothesenkomponenten 12, 13, 14. Die Endoprothesenkomponenten 12, 13, 14 sind untereinander und mit dem Kopfstück 11 ebenfalls über Schraubverbindungen verbunden. Ein Kniestück 15 bildet eine gelenkige Verbindung zu einem Schaft 16, der dazu bestimmt ist, die Endoprothese mit einem Unterschenkelknochen zu verbinden.

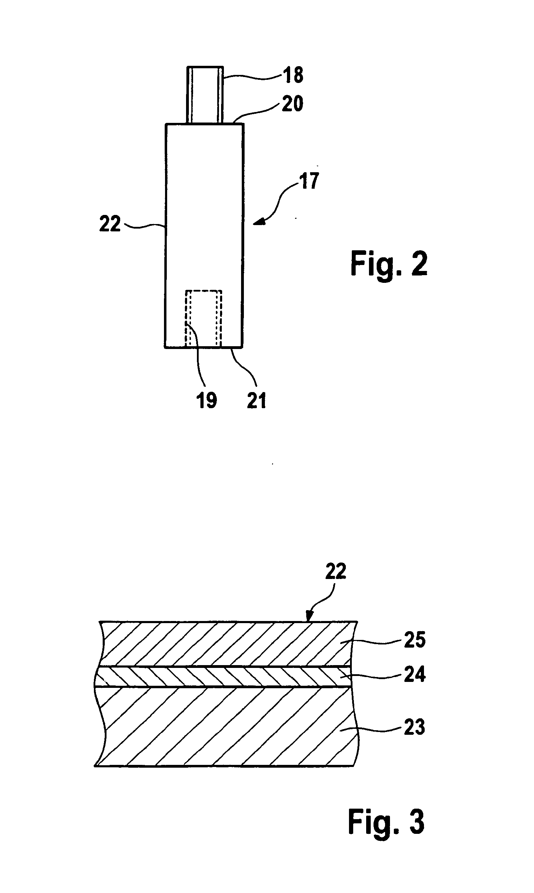

Die Endoprothesenkomponenten 12, 13, 14 sind in unterschiedlichen Längen verfügbar, damit die Endoprothese an unterschiedlich lange Oberschenkelknochen angepasst werden kann. Die Fig. 2 zeigt eine den Endoprothesenkomponenten 12, 13, 14 entsprechende Endoprothesenkomponente 17 in vergrößerter Darstellung. Die Endoprothesenkomponente 17 umfasst einen Schraubbolzen 18 sowie eine gestrichelt angedeutete Schraubbohrung 19. Über den Schraubbolzen 18 und die Schraubbohrung 19 kann die Endoprothesenkomponente 17 an ihren beiden Enden mit gleichartigen Endoprothesenkomponenten, wie etwa den Endoprothesenkomponenten 12, 13, 14, verbunden werden. Der Schraubbolzen 18, die Schraubbohrung 19 sowie die angrenzenden Stirnflächen 20 und 21 bilden also Bereiche an der Oberfläche der Endoprothesenkomponente 17, die dazu ausgelegt sind, mit anderen Endoprothesenkomponenten zusammenzuwirken. Die Mantelfläche 22 der Endoprothesenkomponente 17 hingegen ist dazu ausgelegt, im implantierten Zustand Kontakt mit menschlichem Gewebe einzugehen.

Im Bereich der Mantelfläche 22 ist der die Form der Endoprothesenkomponente 17 vorgebende Körper 23 mit einer Zwischenschicht 24 sowie einer Außenschicht 25 belegt. Die aus Silber bestehende Zwischenschicht 24 belegt den Körper 23 im Bereich der Mantelfläche 22 flächendeckend. Die die Zwischenschicht 24 bedeckende Außenschicht 25 ist eine Hartstoffbeschichtung, die mittels eines Plasmabeschichtungsverfahrens aus Titan, Stickstoff und Silber erzeugt wurde.

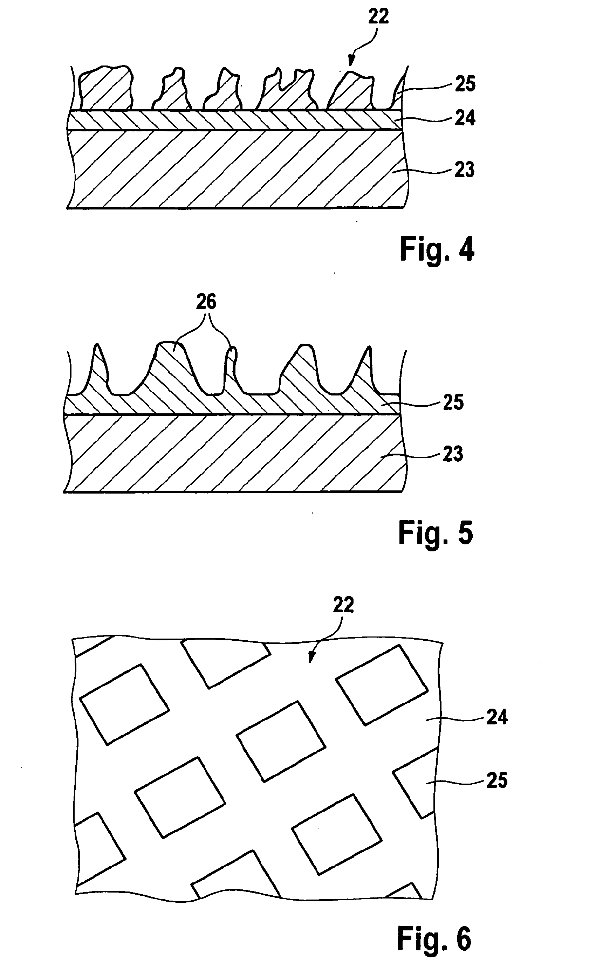

Bei dem in Fig. 4 gezeigten Ausführungsbeispiel ist die Außenschicht 25 porös. Durch mikroskopische Durchbrechungen in der Außenschicht 25 hindurch kann die Zwischenschicht 24 antimikrobiell auf das umgebende Gewerbe wirken.

Fig. 6 zeigt eine Draufsicht auf die Mantelfläche 22 der Endoprothesenkomponente 17 in vergrößerter Darstellung. Die Zwischenschicht 24 ist nur inselförmig mit der Außenschicht 25 belegt. Die Außenschicht 25 hat also makroskopische Durchbrechungen, durch die das umgebende Gewebe in direkten Kontakt mit der Zwischenschicht 24 treten kann. In den Bereichen, in denen die Außenschicht 25 auf die Zwischenschicht 24 aufgebracht ist, kann die Zwischenschicht 24 wie in Fig. 3 gezeigt dicht sein, so dass kein direkter Kontakt zwischen dem umgebendenden Gewebe und der Zwischenschicht 24 besteht.

In Fig. 5 hat die Außenschicht 25 eine Nanostruktur mit ausgeprägten Spitzen 26. Durch elektrisch leitfähige Spitzen 26 bildet sich ein elektrisches Feld um die Spitzen 26 herum, durch das die antimikrobielle Wirkung der Außenschicht 25 verstärkt wird. Die Zwischenschicht ist in Fig. 5 nicht dargestellt.

## Patentansprüche

1. Endoprothesenkomponente, umfassend einen die Form der Endoprothesenkomponente (17) vorgebenden Körper (23), bei der der Körper (23) auf Oberflächenanteilen (22), mit denen die Endoprothesenkomponente im implantierten Zustand in Kontakt mit menschlichem Gewebe steht, mit einer Außenschicht (25) belegt ist, die ein Nitrid, ein Oxinitrid oder ein Oxid auf der Basis eines Refraktärmetalls umfasst, wobei in der Außenschicht (25) Silber und/oder Kupfer enthalten ist, **dadurch gekennzeichnet, dass** zwischen der Außenschicht (25) und dem Körper (23) eine Zwischenschicht (24) angeordnet ist und dass Teile der Zwischenschicht (24) von außen zugänglich sind.

2. Endoprothesenkomponente nach Anspruch 1, **dadurch gekennzeichnet, dass** die Zwischenschicht (24) aus Silber, Kupfer und/oder Gold besteht.

3. Endoprothesenkomponente nach 1 oder 2, **dadurch gekennzeichnet, dass** die Außenschicht (25) durchbrochen ist und dass die Durchbrechungen einen Flächenanteil von mindestens 20%, vorzugsweise mindestens 40%, weiter vorzugsweise mindestens 60% einnehmen.

4. Endoprothesenkomponente nach einem der Ansprüche 1 bis 3, **dadurch gekennzeichnet, dass** die Außenschicht (25) porös ist.

5. Endoprothesenkomponente nach einem der Ansprüche 1 bis 4, **dadurch gekennzeichnet, dass** die Außenschicht (25) eine Mikro- oder Nanostruktur mit ausgeprägten Spitzen (26) hat.

6. Verfahren zum Herstellen einer Endoprothesenkomponente (17) mit den Schritten:
a. Bereitstellen eines die Form der Endoprothesenkomponente vorgebenden Körpers (23), wobei für eine Verbindung mit menschlichem Gewebe ausgelegte Oberflächenateile (22) des Körpers (23) mit einer Zwischenschicht (24) versehen sind;
b. Bereitstellen eines Refraktärmetalls sowie von Silber und/oder Kupfer in Form eines oder mehrerer Targets;
c. Herauslösen von Ionen des Refraktärmetalls sowie von Ionen des Silbers und/oder Kupfers aus dem einen oder den mehreren Targets;
d. Leiten der Ionen zusammen mit einem reaktiven Gas auf mit der Zwischenschicht (24) belegte Oberflächenanteile (22) des Körpers (23), so dass sich eine Außenschicht (25) bildet, durch die hindurch die Zwischenschicht (24) zugänglich ist.

7. Verfahren nach Anspruch 6, **dadurch gekennzeichnet, dass** die Ionen mittels Lichtbogen aus dem einen oder der Mehrzahl von Targets herausgelöst werden.

8. Verfahren nach Anspruch 6, **dadurch gekennzeichnet, dass** die Ionen mittels Laser aus dem einen oder der Mehrzahl von Targets herausgelöst werden.

9. Verfahren nach Anspruch 6, **dadurch gekennzeichnet, dass** die Ionen mittels eines Elektronenstrahls aus dem einen oder der Mehrzahl von Targets herausgelöst werden.

10. Verfahren nach einem der Ansprüche 6 bis 8, **dadurch gekennzeichnet, dass** in Schritt a) ein Körper (23) bereitgestellt wird, bei dem die Zwischenschicht(24) aus Silber, Gold und/oder Kupfer besteht.

11. Verfahren nach einem der Ansprüche 6 bis 9, **dadurch gekennzeichnet, dass** in Schritt c) zusätzlich Bruchstücke mit einem Durchmesser von mehr als 20 µm aus dem Target herausgelöst werden und dass die Bruchstücke in Schritt d) auf den Körper (23) geleitet werden.

12. Verfahren nach einem der Ansprüche 6 bis 10, **dadurch gekennzeichnet**, das vor Schritt d) der zu beschichtende Oberflächenanteil des Körpers mit einer Maske aus einem temperaturstabilen Material belegt wird.

## Claims

1. Endoprosthesis component which comprises a body (23) redefining the shape of the endoprosthesis component (17) and in which, on surface portions (22) with which the endoprosthesis component in the implanted state is in contact with human tissue, the body (23) is covered with an outer layer (25) which comprises a nitride, an oxynitride or an oxide based on a refractory metal, the outer layer (25) containing silver and/or copper, **characterized in that** an intermediate layer (24) is arranged between the outer layer (25) and the body (23) and **in that** parts of the intermediate layer (24) are accessible from the outside.

2. Endoprosthesis component according to Claim 1, **characterized in that** the intermediate layer (24) is composed of silver, copper and/or gold.

3. Endoprosthesis component according to Claim 1 or 2, **characterized in that** the outer layer (25) is interrupted, and **in that** the interruptions occupy a proportion of the surface area of at least 20%, preferably of at least 40%, more preferably of at least 60%.

4. Endoprosthesis component according to one of Claims 1 to 3, **characterized in that** the outer layer (25) is porous.

5. Endoprosthesis component according to one of Claims 1 to 4, **characterized in that** the outer layer (25) has a microstructure or nanostructure with distinct peaks (26).

6. Method for producing an endoprosthesis component (17), with the following steps:
a. Making available a body (23) that predefines the shade of the endoprosthesis component, surface portions (22) of the body (23), which are intended for a connection with human tissue, being provided with an intermediate layer (24);
b. Making available a refractory metal and also silver and/or copper in the form of one or more targets;
c. Releasing ions of the refractory metal and also ions of the silver and/or copper from the one or more targets;
d. Conducting the ions, together with a reactive gas, onto surface portions (22) of the body (23) covered with the intermediate layer (24), so that an outer layer (25) forms through which the intermediate layer (24) is accessible.

7. Method according to Claim 6, **characterized in that** the ions are released from the one or more targets by means of an electric arc.

8. Method according to Claim 6, **characterized in that** the ions are releases from the one or more targets by means of laser.

9. Method according to Claim 6, **characterized in that** the ions are released from the one or more targets by means of an electron beam.

10. Method according to one of Claims 6 to 8, **characterized in that**, in step a), a body (23) is made available in which the intermediate layer (24) consists of silver, gold and/or copper.

11. Method according to one of Claims 6 to 9, **characterized in that**, in step c), fragments with a diameter of more than 20 µm are additionally released from the target, and **in that**, in step d), the fragments are conducted onto the body (23).

12. Method according to one of Claims 6 to 10, **characterized in that**, before step d), the surface portion of the body to be coated is covered with a mask made of a temperature-stable material.

## Revendications

1. Composant pour endoprothèse, comprenant un corps (23) qui définit la forme du composant (17) d'endoprothèse,
des parties (22) de la surface du corps (23) par lesquelles le composant d'endoprothèse est en contact avec le tissu humain lorsqu'il est implanté étant revêtues d'une couche extérieure (25) qui contient un nitrure, un oxynitrure ou un oxyde d'un métal réfractaire,
la couche extérieure (25) contenant de l'argent et/ou du cuivre,
**caractérisé en ce que**
une couche intermédiaire (24) est disposée entre la couche extérieure (25) et le corps (23) et
**en ce que** des parties de la couche intermédiaire (24) sont accessibles depuis l'extérieur.

2. Composant pour endoprothèse selon la revendication 1, **caractérisé en ce que** la couche intermédiaire (24) est constituée d'argent, de cuivre et/ou d'or.

3. Composant pour endoprothèse selon les revendications 1 ou 2, **caractérisé en ce que** la couche extérieure (25) est perforée et **en ce que** les perforations occupent une proportion d'au moins 20 %, de préférence d'au moins 40 % et de manière encore plus préférable d'au moins 60 % de la surface.

4. Composant pour endoprothèse selon l'une des revendications 1 à 3, **caractérisé en ce que** la couche extérieure (25) est poreuse.

5. Composant pour endoprothèse selon l'une des revendications 1 à 4, **caractérisé en ce que** la couche extérieure (25) présente une microstructure ou nanostructure à pointes (26) accusées.

6. Procédé de fabrication d'un composant d'endoprothèse (17), le procédé comportant les étapes qui consistent à :
a. préparer un corps (23) qui définit la forme du composant d'endoprothèse, des parties (22) de la surface du corps (23) prévues pour être reliées au tissu humain étant dotées d'une couche intermédiaire (24),
b. préparer un métal réfractaire ainsi que de l'argent et/ou du cuivre sous la forme d'une ou plusieurs cibles,
c. arracher des ions du métal réfractaire ainsi que des ions d'argent et/ou de cuivre de la ou des cibles,
d. conduire les ions en même temps qu'un gaz réactif sur les parties (22) de la surface du corps (23) occupées par la couche intermédiaire (24) de manière à former une couche extérieure (25) à travers laquelle la couche intermédiaire (24) est accessible.

7. Procédé selon la revendication 6, **caractérisé en ce que** les ions sont arrachés de la ou des cibles au moyen d'un arc lumineux.

8. Procédé selon la revendication 6, **caractérisé en ce que** les ions sont arrachés de la ou des cibles au moyen d'un laser.

9. Procédé selon la revendication 6, **caractérisé en ce que** les ions sont arrachés de la ou des cibles au moyen d'un faisceau d'électrons.

10. Procédé selon l'une des revendications 6 à 8, **caractérisé en ce qu'**à l'étape a), on prépare un corps (23) dont la couche intermédiaire (24) est constituée d'argent, d'or et/ou de cuivre.

11. Procédé selon l'une des revendications 6 à 9, **caractérisé en ce qu'**à l'étape c), on arrache de la cible des fragments d'un diamètre supérieur à 20 µm et **en ce qu'**à l'étape d), les fragments sont amenés sur le corps (23).

12. Procédé selon l'une des revendications 6 à 10, **caractérisé en ce qu'**avant l'étape d), la partie de la surface du corps qui doit être recouverte est occupée par un masque en matériau présentant une bonne tenue à la chaleur.
